# EUROPEAN PATENT SPECIFICATION

(11) **EP 4 153 041 B1**
(45) Date of publication and mention of the grant of the patent: **13.11.2024**
(21) Application number: 21762831.2
(22) Date of filing: 19.07.2021
(51) Int. Cl.: A61B 5/0205, A61B 5/00, A61B 5/0531

(54) **SYSTEM AND METHODS FOR DETECTION AND REMOVAL OF ELECTRICAL NOISE IN DRY ELECTRODES**
SYSTEM UND VERFAHREN ZUR ERKENNUNG UND ENTFERNUNG VON ELEKTRISCHEM RAUSCHEN BEI TROCKENEN ELEKTRODEN
SYSTÈME ET PROCÉDÉS DE DÉTECTION ET D'ÉLIMINATION DE BRUIT ÉLECTRIQUE DANS DES ÉLECTRODES SÈCHES

(43) Date of publication of application: 29.03.2023
(73) Proprietor: Fitbit LLC, San Francisco, CA 94105 (US)
(72) Inventor: BERREITTER, Pieris, San Francisco, CA 94105 (US)
(74) Representative: Maikowski & Ninnemann Patentanwälte Partnerschaft mbB
(86) International application number: PCT/US2021/042183
(87) International publication number: WO 2023/003532

(56) References cited:
- WO-A1-2014/163443
- CA-A1- 2 918 594
- US-A1- 2016 000 344
- US-A1- 2021 106 257
- US-B2- 10 362 940

## Description

### FIELD

The present disclosure relates generally to noise reduction for signals in wearable computing systems.

### BACKGROUND

User computing devices have increased in processing power and utility. User computing devices can include wearable computing devices. One key use for wearable computing devices is measuring user information, such as heart rate, temperature, and so on. This information can be measured using an electrode in contact with the user's skin. In addition, no gel electrolyte can generally be used because of the potential inconvenience to users. As a result, the signal measured by the electrode can potentially have a significant amount of noise introduced that makes it difficult for the user computing device to accurately measure user information. Document CA 2 918 594 A1 discloses a physiological signal extractor comprising a motion artifact reduction unit that can be disposed in or attached to a wearable device. Further, reference US 10 362 940 B2 describes using a differential amplifier in conjunction with an ECG electrode to cancel out noise. Moreover, US 2016/000344 A1 discloses an implantable pressure sensor device for measuring a physiological signal comprising a reference capacitor positioned within the vicinity of a sensing capacitor in order to accurately cancel out noise signals or other artefacts. And document WO 2014/163443 A1 discloses an electronic stethoscope apparatus having a noise removing part for removing sensed noised from sensed bioacoustics.

### SUMMARY

The invention is defined by the independent claims. Aspects and advantages of embodiments of the present disclosure will be set forth in part in the following description, or may be learned from the description, or may be learned through practice of the embodiments. Other example aspects of the present disclosure are directed to systems, apparatus, computer program products (such as tangible, non-transitory computer-readable media but also such as software which is downloadable over a communications network without necessarily being stored in non-transitory form), user interfaces, memory devices, and electronic devices for implementing and utilizing user computing devices.

These and other features, aspects and advantages of various embodiments will become better understood with reference to the following description and appended claims. The accompanying drawings, which are incorporated in and constitute a part of this specification, illustrate embodiments of the present disclosure and, together with the description, serve to explain the related principles.

### BRIEF DESCRIPTION OF THE DRAWINGS

Detailed discussion of embodiments directed to one of ordinary skill in the art are set forth in the specification, which refers to the appended figures, in which:
FIG. 1 illustrates an example computing environment including a user computing device in accordance with example embodiments of the present disclosure.
FIG. 2 illustrates an example noise mitigation system that includes a transducer and a dry electrode in accordance with example embodiments of the present disclosure.
FIG. 3 depicts the front view of an example wearable device according to example embodiments of the present disclosure.
FIG. 4 illustrates an example noise mitigation system in accordance with example embodiments of the present disclosure.
FIGS. 5A-5F illustrate a variety of example configurations of a noise mitigation system in accordance with example embodiments of the present disclosure.
FIG. 6 illustrates an example user interface for a smartwatch in accordance with example embodiments of the present disclosure.
FIG. 7 is a flowchart depicting an example process of mitigating noise in a user computing device in accordance with example embodiments of the present disclosure.

### DETAILED DESCRIPTION

Reference now will be made in detail to embodiments, one or more examples of which are illustrated in the drawings. Each example is provided by way of explanation of the embodiments, not limitation of the present disclosure. In fact, it will be apparent to those skilled in the art that various modifications and variations can be made to the embodiments without departing from the scope of the present disclosure. For instance, features illustrated or described as part of one embodiment can be used with another embodiment to yield a still further embodiment. Thus, it is intended that aspects of the present disclosure cover such modifications and variations.

Generally, the present disclosure is directed towards a system for mitigating the effect of signal noise on data captured by sensors included in a user computing device (e.g., such as a fitness band) in contact with skin. To do so, the user computing device includes a physiological signal sensor, such as a dry electrode (e.g., a single metal sensor) in contact with the skin that acts as a conductor between the skin and the user computing device. The dry electrode is the source of a first signal used by the user computing device in any one of a plurality of analysis algorithms. The first signal can represent a signal generated based on one or more physiological phenomenon. The user computing device also includes a transducer. The transducer can include any of a plurality of different sensor types (e.g., a microphone, a pressure sensor, a capacitance sensor, and so on) that can measure variations in a physical quality (e.g., pressure, sound, brightness) and cover those variations into an electrical signal. The transducer can also be referred to as a noise sensor. The transducer can measure deviations in pressure or translational movement of the electrode against the skin. The signal produced by the transducer can then be used to modify the first signal (e.g., summing, subtracting, or comparing the two signals). In some examples, portions of the first signal may be flagged to be ignored by the user computing device because excessive noise may make the signal at the moment unreliable. The modified first signal can then be used as input to a plurality of algorithms to generate analysis data about a user. In the context of the claimed invention, the transducer includes an audio sensor.

In a simple example, the wearable device may be a smartwatch. The smartwatch can include the capability to estimate a user's heart rate (or other information about a user's heart health) based on electrocardiograph data gathered or estimated based on a dry electrode sensor included in the smartwatch. For example, a user seeking to determine their heart rate can be instructed to place two fingers on one or more sensors included in the smartwatch (e.g., a metal ring around the face of the watch). The signal detected from the fingers can be affected, at least in part, by the pressure applied by the user while the data from which the heart rate will be estimated is gathered. For example, if the user changes the pressure with which they contact their fingers to the one or more dry electrode sensors, the change in pressure can result in inconsistencies with the captured data (i.e., signal noise) that can cause the data to be difficult to interpret.

To overcome this signal noise, the smartwatch can include one or more pressure sensors located such that they can detect the amount of pressure being applied by the user as they place their fingers on the one or more dry electrode sensors. The pressure sensors can produce a noise signature signal (e.g., based on the amount of pressure being applied) that can represent the amount of change in the pressure applied by the user (e.g., whether it increases or decreases). The noise signature signal generated by the pressure sensors can then be used to adjust the first signal received from the dry electrodes. For example, if the pressure increases, the resulting first signal can be manipulated or modified such that the signal represents the value that would have been generated if the pressure had been constantly maintained. In some examples, if the pressure sensor(s) determine that the pressure has changed more than a threshold value, the smartwatch can prompt the user to adjust the pressure with which they are placing their fingers on the one or more sensors to achieve a more accurate reading. The modified first signal can then be analyzed to provide an estimate of the user's heart rate that is more accurate than the unmodified first signal.

More specifically, a user computing device can include any computing device that it used by a user. User computing devices can include personal computers, laptops, smartphones, tablet computers, wearable computing devices, and so on. A wearable computing device can be any computing device that is integrated into an object that is meant to be worn by a user. For example, wearable computing devices can include, but are not limited to smartwatches, fitness bands, computing devices integrated into jewelry such as smart rings or smart necklaces, computing devices integrated into items of clothing such as jackets, shoes, and pants, and wearable glasses with computing elements included therein. The user computing device can also include a home testing unit that a user can use to test one or more physiological characteristics. In some examples, a user computing device can include one or more sensors intended to gather information with the permission of the user of the user computing device. Such information can include, but is not limited to, heart rate, body composition, sweat level, hydration level, and so on

In some examples, the user computing device can include one or more physiological signal sensors. A physiological signal sensor can be a dry electrode. In the context of the claimed invention, the user computing device includes a dry electrode. A dry electrode can consist of a single type of metal (e.g., as opposed to a combination of metals as may be more commonly used in wet electrodes) that is in direct contact with the skin of a user. The dry electrode can act as a conductor between the skin of the user and the electrode without the need for a mediating agent. In contrast, a wet electrode uses an electrolyte gel as a conductor between the skin and the electrode. The dry electrode therefore can allow data to be gathered from the skin of a user without the need to apply any electrolyte gel to the skin of the user. In some examples, a user can generate bioelectrical or electrophysiological signals (e.g., generated by cells or tissues within the user) that can be detected by the dry electrode. The signal measured by the dry electrode can be referred to as the first signal. The first signal can be measured and recorded in the form of electroencephalograms, electrocardiograms, electromyography, electrooculograms, etc.

Because dry electrodes do not use electrolyte gel, the signals produced may experience greater signal noise than the signals produced by wet electrodes. The signal noise includes distortions of the first signal generated by the dry electrode based on one or more factors that can distort the signal. The signal distortion can be introduced from a variety of different sources. For example, translational movement of the dry electrode along the skin (e.g., the sensor moving back and forth), increased or decreased pressure, increased or decreased capacitance, and so on. According to an example aspect of the present disclosure, in order to identify and remove signal noise that would result in incorrect readings, the user computing device can include a transducer that measures possible sources of noise such as pressure or translational movement, which can manifest as noise in the first signal. In some examples, the distortion of the first signal can result in reduction in the signal quality of the first signal. For example, if the pressure applied by a user decreases during the measurement of the first signal, the quality of the first signal may degrade. The portion of the first signal with altered quality may be marked or flagged to distinguish it from other portions of the first signal.

In some examples, the sensor can be a transducer that measures one of a variety of factors including but not limited to noise, movement, changes in pressure, and changes in capacitance and produces a noise signal that represents the amount, degree, or intensity of the measured noise factor at a point in time. The noise signal can be analyzed and processed such that it can be used to modify the first signal measured by the dry electrode. For example, the noise signal can be subtracted from the first signal either in the temporal domain or in the frequency domain. In some examples, the transducer can be an audio sensor such as a microphone. The audio sensor can measure audio signals and use that as a representation of the noise experienced by the first signal at a given point in time. The audio sensor can be incorporated into the user computing device in a plurality of different configurations depending on one or more different factors.

For example, the audio sensor is configured to measure audio signals inside the case of the user computing device. A measurement of the audio signals inside of the case of a user computing device can represent noise introduced by the translational movement of the user computing device along the skin of the user. Translational movement of the user computing device can result in sounds signals being generated by the skin rubbing against the case of the user computing device. These sound signals can be detected on the interior of the case of the user computing device, while other external sound signals (e.g., any sounds outside of the user computing device) are significantly reduced. Thus, the audio sensor configured to sense audio signals on the inside of the case can measure noise introduced by the translational movement of the device.

In another configuration, an audio sensor can be configured such that it is ported to the outside of the user computing device. In this way, the audio sensor can determine whether audio disturbances are happening in the area of the user computing device. In addition, the audio sensor may also detect sound signals generated by translational motion as well when ported to the outside environment.

In some examples, the transducer can be a pressure sensor that can detect an amount of pressure caused by the contact of a user's finger on a sensor of the user computing device. For example, a user computing device may instruct the user to place their fingers on two contacts on the user computing device while a measurement is being taken. During this time, the degree of pressure that the user uses can vary. Differences in pressure can result in amplitude variation (e.g., noise) in the first signal being measured by the device. For example, increasing or decreasing pressure can change the surface area in contact with the dry electrode and thus alter the detected signal.

In this example, a pressure sensor can be used to determine how much pressure the user is applying while touching their fingers to the dry electrode. The variation in pressure can be recorded as the noise signature signal and used by the user computing device to correct irregularities in the first signal.

Similarly, the transducer can be a capacitance sensor and can detect changes in surface contact area that reflect possible changes in the contact resistance measured in the first signal. As with pressure sensors and audio sensors, this data can be used to generate a noise signature signal which can be used to correct irregularities in the first signal.

In some examples, the transducer can be a strain gauge. The strain gauge can be a sensor whose resistance varies according to an applied force. The resistance can be measured and used to estimate the amount of strain applied to the user computing device. Strain applied to the user computing device can be a source of noise and as such, measuring the strain can allow a noise mitigation system to mitigate any noise introduced as a result of the strain applied to the user computing device.

Once the transducer has detected the noise signature signal, the signal can be processed. For example, the noise signature signal can be amplified, filtered, and characterized. In some examples, the noise signature signal can be translated into a specific domain. For example, the user computing device can translate the detected noise signature signal into the time domain and or the frequency domain as needed.

The user computing device can modify the first signal based on the noise signature signal. In some examples, this modification can include subtracting elements associated with the noise signature signal from the first signal. In this way, if the first signal has been distorted to be greater than the signal would be without noise, the noise signature signal can be subtracted from the first signal such that the resulting modified first signal is a better representation of the actual underlying data than the original first signal.

In some examples, modifying the first signal can include adding elements of the noise initial signal to the first signal. For example, if the noise signature signal is based on a measurement of pressure applied by a user, the user may reduce the pressure applied over time. In this example, the user computing device can modify the first signal by adding the noise signature signal (or elements of the noise signature signal) to the first signal to create the modified first signal. Note that in some examples, rather than adding the noise signature signal to the first signal, the user computing device can increase the gain of the first signal by an amount associated with the noise signature signal. Similarly, the user computing device can compare the first signal to the noise signature signal to determine whether or not the first signal should be modified. In some examples, the first signal and the noise signature signal have associated timestamps that can be used to accurately compare the same time in each signal.

In some examples, rather than directly modifying the first signal, the user computing device can designate one or more sections of the first signal as noisy and those sections can be disregarded when generating information about the user. For example, a noise mitigation system in the user computing device can determine whether, at a particular point in time, the noise level exceeds a predetermined noise threshold. If the noise mitigation system determines that the noise level exceeds the predetermined threshold, the noise mitigation system can generate a noise flag for that specific period of time. In some examples, the noise mitigation system can determine when the noise value falls below the predetermined threshold and generate a flag marking the end of the noisy section.

In that example, the wearable computing system can disregard information from the first signal during the time between a first flag designating the beginning of the noisy section and a second flag designating the end of the noisy section. In some examples, the noise mitigation system can generate a data set for each period of time of the noise signature signal. Thus, each period of time (e.g., in 100 millisecond increments) can have an associated value that indicates whether or not the noise during that period of time exceeds a predetermined threshold value (e.g., a Boolean value). The wearable computing system can then use the data set to determine which periods of time in the first signal should be ignored.

In another example, the noise mitigation system can generate flags that indicate the beginning and ending portions of the noise signature signal that exceed the predetermined threshold value. Thus, the first flag can be generated to represent a timestamp of the beginning of a portion of the noise signature system that exceeds a predetermined threshold value and the second flag can be generated to represent a timestamp that represents the ending of the portion of the noise signature signal that exceeds a predetermined threshold value. The user computing device can then use these flags to exclude the designated portions of the first signal from consideration.

The user computing device can determine whether sufficient data has been received via the modified first signal. For example, the user computing device can determine that 30 seconds of data is necessary to estimate a stress level associated with the user. The user computing device can monitor the modified first signal data to determine whether 30 seconds of data has been received. If a significant number of data points are ignored based on noise levels (e.g., some data may be excluded based on noise levels as explained above), the user computing device can present a notification to the user (e.g., on add display in the watch face) that additional time is needed and present a countdown for the user.

In some cases, the user computing device can determine, based on the noise signature signal, whether the user alters one or more aspects of the input they are giving (e.g., the amount of pressure, the area of contact, the amount of translational movement). In one example, if the user increases the pressure that they are applying during the measurement time, the user computing device can determine that some of the first signal data is unusable. The user computing device can present a request to the user to decrease the pressure level of the contact with the dry electrode or to minimize translational movement.

Similarly, in accordance with a determination that at least one or more portions of the first signal are ignored, the user computing device can determine any additional amount of data that needs to be gathered to estimate particular data about a user. For example, if 30 seconds of data is needed to estimate a heart rate for a user, and the first signal has generated 15 seconds of usable data, the user computing device can determine that 15 more seconds of data is required. If so, the wearable computing system can display a notification to the user requesting that the user continue contacting the dry electrode for the remaining amount of time.

Once the first signal has been modified, the user computing device can use the data contained therein to perform one or more algorithms. A variety of algorithms can be performed to estimate a variety of different attributes of the user. For example, data gathered through the first electrode can be used to generate an electrocardiograph. The electrocardiograph can be a graph of voltage versus time and can represent the electrical activity of the user's heart. In some examples, this data can be used to determine or estimate the user's heart rate, to identify irregularities with the user's heart rhythm, heart blockages and conduction problems, electrolyte disturbances, and so on.

In another example, the user computing device can perform bioelectric impedance analysis. In some examples, user computing devices can use this data to estimate the composition of a user's body.

The following provides an end-to-end example of the technology described herein. A user computing device can include a first electrode and a transducer. In some examples, the user computing device generates, by a first electrode in contact with a user's skin, a first signal that represents one or more characteristics associated with a user. In the context of the claimed invention, the physiological signal sensor is a dry electrode in contact with a user's skin. For example, the dry electrode can be a single metal contact that, when placed in contact with the user's skin, can generate a first signal in response to one or more characteristics of the user's skin or body.

The user computing device can generate a noise signature signal via a transducer. In some examples useful to understand the claimed invention, the transducer is an audio sensor situated to detect audio signals internal to the user computing device generated by lateral movement of the user computing device along the skin of the user. In another example, the transducer is a pressure sensor configured to determine variations in the pressure applied by a user.

In some examples, the transducer can be a capacitance sensor and the noise signature signal can represent changes in the area in which changes in differences in effective capacitance are detected. In some examples useful to understand the claimed invention, the transducer is an accelerometer and the noise signature signal represents a movement of the user computing device.

The user computing device can modify the first signal based on the noise signature signal to generate a modified first signal. The first signal and the noise signature signal can be represented in one of the time domain or the frequency domain.

In some examples, modifying the first signal involves subtracting the noise signature signal from the first signal to generate the modified first signal. In other examples, modifying the first signal can comprise determining, at a particular time, whether the noise signature signal exceeds a predetermined threshold value. In accordance with a determination that the noise signature exceeds the predetermined threshold value, the user computing device stores a timestamp of the particular time. The user computing device can exclude the first signal associated with the timestamp from consideration when determining one or more characteristics associated with a user based on the modified first signal.

The user computing device can utilize a predetermined amount of time associated with gathering sufficient data to determine one or more characteristics associated with the user. In some examples, before the predetermined amount of time has elapsed, the user computing device can determine that the modified first signal includes data sufficient to determine one or more characteristics associated with the user. For example, if the user computing device is estimating the heart rate of a user, the user computing device can determine that it has enough data to estimate the heart rate of a user before the predetermined amount of time has already elapsed.

In some examples, the user computing device can present, on a display associated with the user computing device, a notification that the user can disengage from contact with the dry electrode.

Similarly, the user computing device can utilize a predetermined amount of time associated with gathering sufficient data to determine one or more characteristics associated with the user. When the predetermined amount of time has elapsed, the user computing device can determine that the modified first signal does not include data sufficient to determine one or more characteristics associated with the user. The user computing device can present, on a display associated with the user computing device, a request that the user continues to remain in contact with the dry electrode.

In some examples useful to understand the claimed invention, the transducer is a pressure sensor. The user computing device can detect, using the transducer based on the noise signature signal, a change in pressure applied by the user. The user computing device can determine that the change in pressure applied by the user exceeds a predetermined threshold. In accordance with a determination that the change in pressure applied by the user exceeds the predetermined threshold, the user computing device can display a message to the user requesting the user to alter the pressure applied.

In some examples, the user computing device can determine one or more characteristics associated with a user based on the modified first signal. The one or more characteristics associated with the user include the presence of sweat on a surface of the user's skin. In some examples, the presence of sweat on the surface of the user's skin is used to estimate a stress level associated with the user.

In some examples, the one or more characteristics associated with the user includes an impedance value as measured between two points on the user's skin. The impedance value as measured between two points on the user's skin can be used to estimate a stress level associated with the user.

Embodiments of the disclosed technology provide a number of technical effects and benefits, particularly in the areas of user computing devices. In particular, embodiments of the disclosed technology provide improved techniques for mitigating noise that may occur in the first signal generated by a dry electrode. For example, utilizing embodiments of the disclosed technology, a user computing device can more accurately determine one or more physiological characteristics of a user (e.g., heart rate, body composition, and so on). Effectively mitigating noise and increasing the accuracy of estimation can result in a better and more useful user experience. Furthermore, this effect is accomplished with relatively little cost. As such, the disclosed embodiments enable additional functionality without significantly increasing the total cost of a wearable device.

With reference now to the figures, example aspects of the present disclosure will be discussed in greater detail.

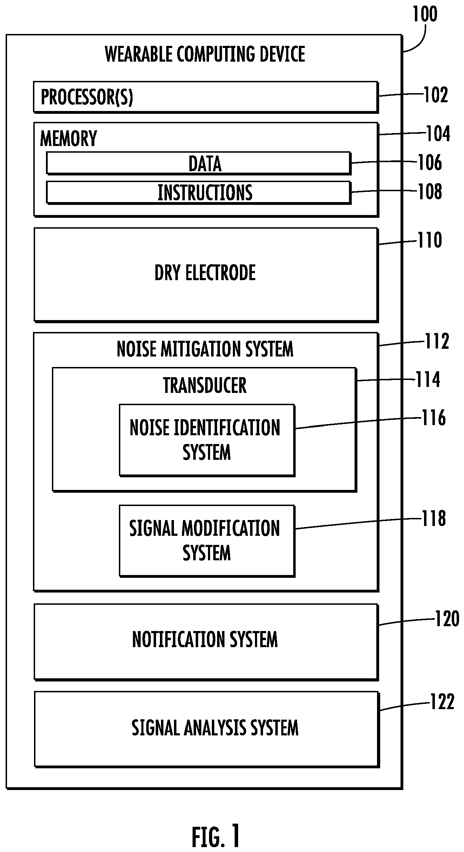

FIG. 1 illustrates an example computing environment including a user computing device 100 in accordance with example embodiments of the present disclosure. In this example, the user computing device 100 can include one or more processors 102, memory 104, a dry electrode 110, a noise mitigation system, 112, and a notification system 120.

In more detail, the one or more processors 102 can be any suitable processing device that can be embedded in the form factor of a user computing device. For example, such a processor can include one or more of: one or more processor cores, a microprocessor, an ASIC, a FPGA, a controller, a microcontroller, etc. The one or more processors can be one processor or a plurality of processors that are operatively connected. The memory 104 can include one or more non-transitory computer-readable storage mediums, such as RAM, ROM, EEPROM, EPROM, flash memory devices, etc., and combinations thereof.

In particular, in some devices, memory 104 can store instructions for implementing the noise mitigation system 112, the noise identification system 116, the signal modification system 118, the notification system 120, and/or the signal analysis system 122. The wearable computing system 100 can implement the signal modification system to execute aspects of the present disclosure, including mitigating noise in the signal of the dry electrode 110.

It will be appreciated that the term "system" can refer to specialized hardware, computer logic that executes on a more general processor, or some combination thereof. Thus, a system can be implemented in hardware, application specific circuits, firmware and/or software controlling a general-purpose processor. In one embodiment, the system can be implemented as program code files stored on the storage device, loaded into memory, and executed by a processor or can be provided from computer program products, for example computer executable instructions, that are stored in a tangible computer-readable storage medium such as RAM, hard disk or optical or magnetic media.

Memory 104 can also include data 106 and instructions 108 that can be retrieved, manipulated, created, or stored by the one or more processor(s) 102. In some example embodiments, such data can be accessed and used as input to the noise mitigation system 112, the signal modification system 118, the notification system 120, or the noise identification system 116. In some examples, the memory 104 can include data used to perform one or more processes and instructions that describe how those processes can be performed.

The user computing device 100 includes a physiological signal sensor. The physiological signal sensor is a dry electrode 110. In contrast, a wet electrode uses an electrolyte gel as a conductor between the skin and the electrode. The dry electrode 110 therefore can allow data to be gathered from the skin of a user without the need to apply any electrolyte gel to the skin of the user. As noted above, a user can generate bioelectrical or electrophysiological signals (e.g., generated by cells or tissues within the user) that can be detected by the dry electrode 110. The signal measured by the dry electrode 110 can be referred to as the first signal. The first signal can be measured and recorded in the form of electroencephalograms, electrocardiograms, electromyography, electrooculograms, etc. It should be noted that the dry electrode can be an active electrode or a passive electrode.

The noise mitigation system 112 can mitigate the noise caused in the first signal detected by the dry electrode 110. As noted above, because dry electrodes do not use electrolyte gel, the signals produced may experience greater signal noise than the signals produced by wet electrodes. According to an example aspect of the present disclosure, in order to identify and remove signal noise that would result in incorrect readings, the user computing device can include a noise mitigation system 112 that can measure and counteract any noise.

In some examples, the noise mitigation system 112 can include a transducer 114, a noise identification system 116, and a signal modification system 118. The transducer 114 can be a sensor that measures one of a variety of factors including but not limited to noise, movement, changes in pressure, and changes in capacitance and produces a noise signal that represents the amount, degree, or intensity of the measured noise factor at a point in time.

The noise signal can be analyzed and processed such that it can be used to modify the first signal measured by the dry electrode 110. In the context of the claimed invention, the transducer 114 includes an audio sensor such as a microphone. More generally, in examples useful to understand the claimed invention, the transducer 114 can be a pressure sensor, a capacitance sensor, a movement sensor (e.g., a gyroscope, an accelerometer, and so on). In the context of the claimed invention, the transducer 114 (e.g., when the transducer 114 is an audio sensor) can measure audio signals and use the measured audio signals as a representation of the noise experienced by the first signal at a given point in time. The transducer 114 sensor can be incorporated into the user computing device 100 in a plurality of different configurations depending on specific type of noise expected for the particular user computing device 100.

Once the transducer 114 has detected the noise signal, the noise identification system 116 can determine whether the noise detected exceeds a predetermined threshold value. For example, the noise mitigation system 112 can be calibrated to determine the level of noise at which the first signal is significantly degraded. The predetermined threshold can be a noise level at which the first signal begins to degrade beyond the point of being useful. In some examples, if the noise level exceeds the threshold, the noise identification system 116 can transmit the noise signal to the signal modification system 118.

The signal modification system 118 can determine, based on the noise signal and the type of sensor used as a transducer, a method for modifying the signal. For example, the noise signal can be subtracted from the first signal, the noise signal can be added to the first signal, the two signals can be compared, or particular time sections of the first signal can be designated to be excluded from the analysis performed by the user computing device 100.

In some examples, the notification system 120 can generate a notification to a user based on the noise signal. For example, if the noise is the result of a user's actions (e.g., the user has increased or reduced pressure on the pressure sensor), the notification system 120 can generate a notification instructing the user to modify their behavior to reduce the noise. In another example, the noise can be the result of movement of the device along the user skin and the notification can be instructions to hold the device still. In some examples, the user computing device 100 has a predetermined amount of the first signal needed to perform or more measurement tasks. The notification system 120 can generate instructions to the user to provide additional data. For example, the wearable computer device 100 can require 30 seconds of first single do that to accurately generate an electrocardiograph. If, as a result of noise in the signal, 10 seconds of first signal data has to be discarded, the notification system 120 can prompt the user to continue contact with the sensor for another 10 seconds.

Once the first signal has been modified, the signal analysis system 122 can use the data contained therein to perform one or more algorithms. A variety of algorithms can be performed to estimate a variety of different attributes of the user. For example, data gathered through the first electrode can be used to generate an electrocardiograph. The electrocardiograph can be a graph of voltage versus time and can represent the electrical activity of the user's heart. In some examples, this data can be used to determine or estimate the user's heart rate, to identify irregularities with the user's heart rhythm, heart blockages and conduction problems, electrolyte disturbances, and so on.

In another example, the signal analysis system 122 can perform bioelectric impedance analysis. In some examples, user computing devices can use this data to estimate the composition of a user's body.

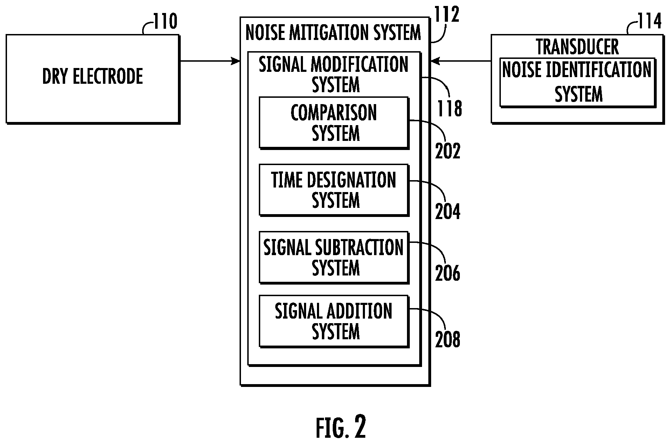

FIG. 2 illustrates an example noise mitigation system 112 that includes a transducer 114 and a dry electrode 110 in accordance with example embodiments of the present disclosure. As noted above, the noise mitigation system 112 can include a signal modification system 118. The system modification system 118 can include a comparison system 202, a time designation system 204, and a signal subtraction system 206, and signal addition system 208.

The comparison system 202 can compare the first signal received from the dry electrode 110 to the noise signal received from the transducer 114. In some examples, the comparison between the two signals can determine how the signal is modified. For example, if the first signal is large relative to the noise signal, the noise signal may be ignored for the period of time in which the first signal is large. Conversely, if the noise signal is large relative to the first signal, the signal modification system can discard the first signal for that period of time. In some examples, the comparison system 202 can determine the type of modification that the signal modification system 118 will perform.

For example, the comparison system 202 can determine whether the noise signal needs to be subtracted from the first signal, whether the noise signal needs to be added to the first signal, or whether the signal modification system 118 should designate one or more time periods as discardable.

In accordance with the determination by the comparison system 202 that the noise signal should be subtracted from this first signal, the signal subtraction system 206 can modify the first signal by subtracting the noise signal from it. Similarly, if the comparison system 202 determines that the noise signal should be added to the first signal, the signal addition system 208 can add the noise signal to the first symbol.

In accordance with a determination by the comparison system 202 that one or more portions of the noise signal should be designated as discardable, the time designation system 204 can generate time series data associated with the level of noise in the noise signal. Each data point in the time series data can represent a discrete period of time relative to the noise signal and the first signal. For example, if each point represents 1/10th of a second, the time series data for the first 10 seconds will include 100 discrete data points. In some examples, each data point can indicate whether or not the corresponding point in the first signal should be retained or discarded. For example, each value can be a Boolean value (e.g., either true or false).

In another example, the time designation system 204 can generate a marker when the noise signal exceeds a predetermined threshold (e.g., when the noise signal is sufficient to degrade the first signal to a predetermined degree) and another marker when the noise signal falls back below the predetermined threshold. Thus, only data representing the beginning and ending of periods in which the noise signal exceeds a predetermined threshold is retained by the signal modification system using this data, the signal modification system 118 can discard portions of the noise signal that are too noisy.

The noise mitigation system 112 can receive a first signal from the dry electrode 110. As noted above, the dry electrode can gather data from the skin of a user by detecting bioelectrical or electrophysiological signals (e.g., generated by cells or tissues within the user). The first signal can represent the bioelectrical or electrophysiological signal transmitted by the dry electrode 110 to the noise mitigation system 112.

The noise mitigation system 112 can receive a noise signal from the transducer 114. The noise signal can be analyzed by the noise of notification system 120 to determine whether the noise level exceeds a predetermined threshold. In some examples, if the noise signal does not exceed a predetermined threshold no signal modification is necessary.

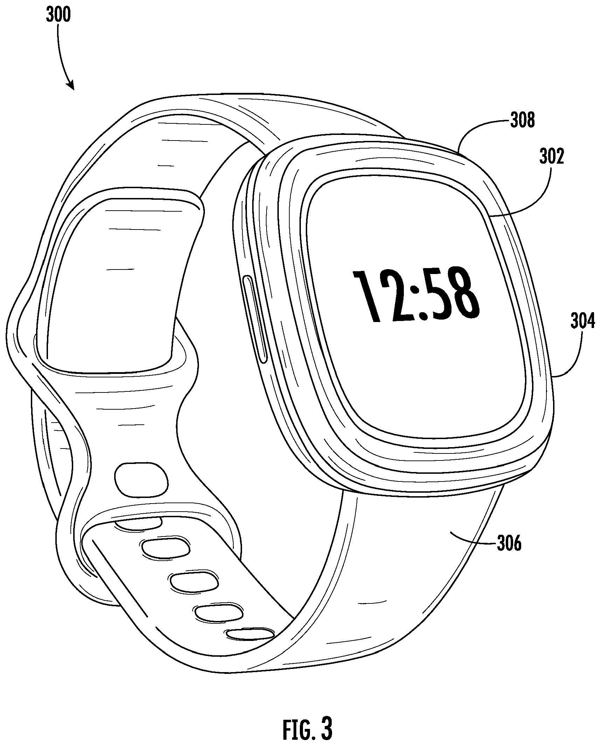

Figure 3 depicts the front view of an example wearable device 300 according to example embodiments of the present disclosure. In an embodiment, the wearable device 300 may be a wristband, a bracelet, a wristwatch, an armband, a ring placed around a digit of the user, or other wearable products that may be equipped with sensors as described in the present disclosure. In an embodiment, the wearable device 300 is configured with a display 302, a device housing 304, a band 306, and one or more additional sensors 308. In an embodiment, the display 302 can be configured to present to a user data relating to the user's skin temperature, heart rate, electroencephalograms, electrocardiograms, electromyography, electrooculograms, and other physiological data of the user (e.g., blood oxygen level). The display 302 can also be configured to convey data from additional ambient sensors contained within the wearable device 300. Example information conveyed on the display 302 from these additional ambient sensors can include a positioning, altitude, and weather of a location associated with the user. The display 302 can also convey data regarding motion of the user (e.g., whether the user is stationary, walking, and/or running).

In an embodiment, the display 302 can be configured to receive data input by the user. In an embodiment, a user can, by input on the display, request that the user computing device 300 generate additional data for display to the user (e.g., electrocardiograph data). In response, the display can present instructions to the user (e.g., instructions to place one or more fingers on sensor 310) to obtain the data requested. Furthermore, if, while the additional data is being gathered, the user computing device 300 determines that additional data is necessary, the display 302 can display instructions to the user (e.g., please continue to hold your finger against the sensor for 10 seconds).

In an embodiment, the device housing 304 can be configured to contain one or more sensors described in the present disclosure. Example sensors contained by the device housing 304 can include skin temperature sensors, internal device temperature sensors, location sensors (e.g., GPS), motions sensors, altitude sensors, heart rate sensors, audio sensors, pressure sensors, and other physiological sensors (e.g., blood oxygen level sensors). In an embodiment, the device housing 304 can also be configured to include one or more processors. The band 306 can be configured to secure the wearable device 100 around an arm the user by, for example, connecting ends of the band 306 with a buckle, clasp, or other similar securing device, thereby allowing the wearable device 100 to be worn by the user.

The one or more additional sensors 308 can include a dry electrode. A dry electrode can allow data to be gathered from the skin of a user without the need to apply any electrolyte gel to the skin of the user. As noted above, a user can generate bioelectrical or electrophysiological signals (e.g., generated by cells or tissues within the user) that can be detected by the dry electrode 110. The signal measured by the dry electrode 110 can be referred to as the first signal. The first signal can be measured and recorded in the form of electroencephalograms, electrocardiograms, electromyography, electrooculograms, etc. It should be noted that the dry electrode can be an active electrode or a passive electrode.

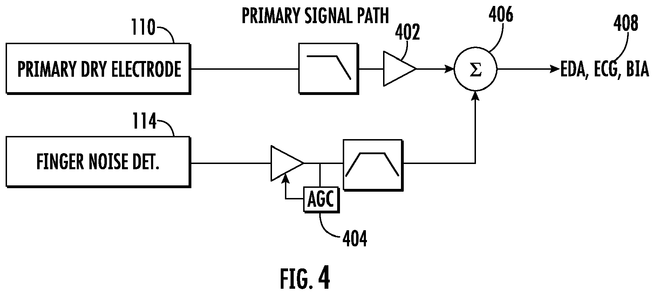

FIG. 4 illustrates an example noise mitigation system (e.g., noise mitigation system 112 in FIG. 1) in accordance with example embodiments of the present disclosure. In this noise mitigation system 112, a dry electrode 110 can capture and/or detect an electrophysiological signal and transmit it along the first signal path 402. In some examples, the first signal path can include signal amplification and/or amplification to clean or improve the first signal.

The transducer 114 can detect a noise signal based on one or more sources of noise (e.g., changes in pressure, changes in capacitance, movement of the sensor, and so on). The noise signal can be amplified and filtered 404. Both the first signal and the noise signal can be transmitted to a signal modifier 406. The signal modifier 406 can modify the signal as discussed above (e.g., subtracted, added, compared, and so on). The signal can be used for one or more measurements 408, including, but not limited to electroencephalograms, electrocardiograms, electromyography, electrooculograms.

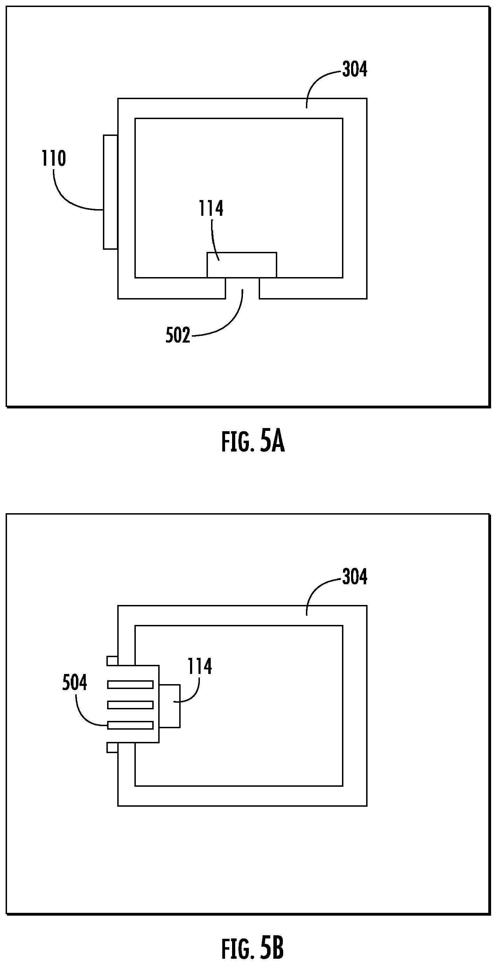

FIGS. 5A-5F illustrates a variety of example configurations of a noise mitigation system in accordance with example embodiments of the present disclosure. FIG. 5A illustrates an example configuration including the housing 304 of the user computing device (e.g., user computing device 100 in FIG. 1), a dry electrode 110, a port 502 from the inside of the housing to the outside, and a transducer 114. In this example configuration the transducer 114 is a microphone (or other audio sensor) that can measure audio signals outside the user computing device 100 through the port 502. The audio signals are associated with noise in the first signal due to translational movement of the dry electrode 110. However, due to the port 502, the transducer 114 may also detect a significant amount of audio noise that is not related to noise in the first signal.

FIG. 5B illustrates an example configuration including the housing 304 of the user computing device (e.g., user computing device 100 in FIG. 1), a port integrated into the dry electrode itself 504, and a transducer 114. In this example configuration, the port is integrated into the dry electrode itself creating an integrated port electrode 504. The port is made using micro-perforations through the electrode into the outside environment. Similar to FIG. 5A, the transducer 114 is a microphone (or other audio sensor) that can measure audio signals outside the user computing device 100 through the integrated port electrode 504. The audio signals are associated with noise in the first signal due to translational movement of the dry electrode 110. However, due to the port 502, the transducer 114 may also detect a significant amount of audio noise that is not related to noise in the first signal.

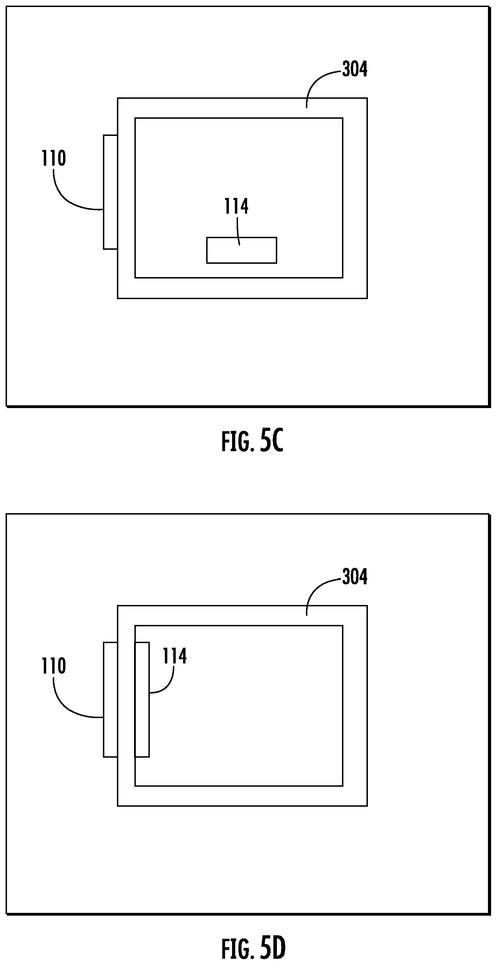

FIG. 5C illustrates an example configuration including the housing 304 of the user computing device (e.g., user computing device 100 in FIG. 1), an electrode 110, and a transducer 114. In this example configuration, there is no port from inside the housing 304. Instead, the dry electrode 110 can measure a signal from outside the housing 304 while the transducer 114 (a microphone or other audio sensor) can measure audio signals inside the housing 304 of the user computing device 100. In this way, audio signals associated with translational movement of the user computing device 100 can be detected from within the housing 304, while other audio signals are less detectable inside the housing 304.

FIG. 5D illustrates an example configuration including the housing 304 of the user computing device (e.g., user computing device 100 in FIG. 1), a dry electrode 110, and a pressure sensor transducer 114. In this example configuration, the transducer 114 is a pressure sensor that can measure differences in pressure applied to the dry electrode 110. The differences in pressure can be associated with noise in the first signal.

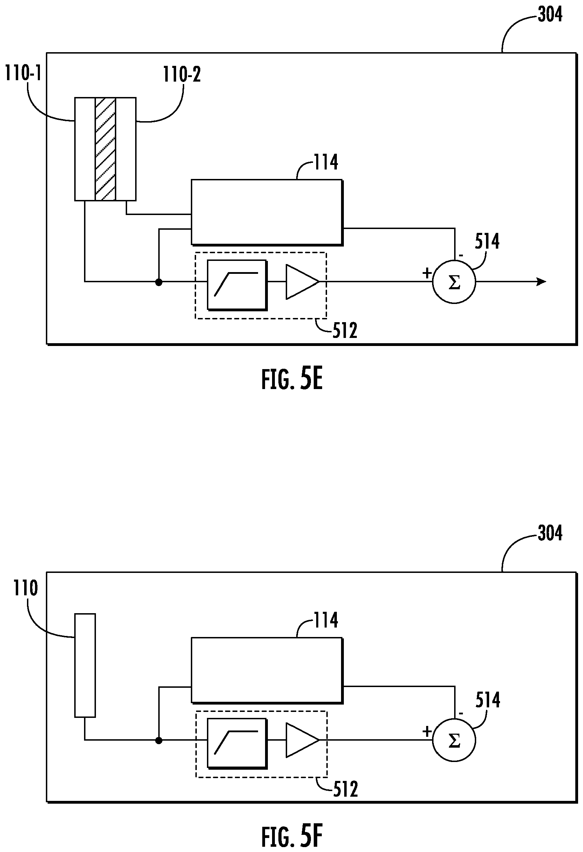

FIG. 5E illustrates an example configuration including the housing 304 of the user computing device (e.g., user computing device 100 in FIG. 1), two electrodes separated by a dielectric, a transducer 114, a signal processing system 512, and a signal modifier 514. In this example configuration, the transducer 114 is a mutual capacitance sensor. The two electrodes can generate a first signal based on a determination of a difference of the mutual coupling between the two electrodes caused by an object (e.g., a finger). The transducer 114 can detect changes in the measured capacitance. The system also includes a signal processing system 512 for filtering and amplifying the first signal.

FIG. 5F illustrates an example configuration including the housing 304 of the user computing device (e.g., user computing device 100 in FIG. 1), an electrode 110, a transducer 114, a signal processing system 512, and a signal modifier 514. In this example configuration, the transducer 114 is a self-capacitance sensor. Similar to FIG. 5E, the dry electrode measures the capacitance as the first signal and the transducer 114 measures changes in capacitance.

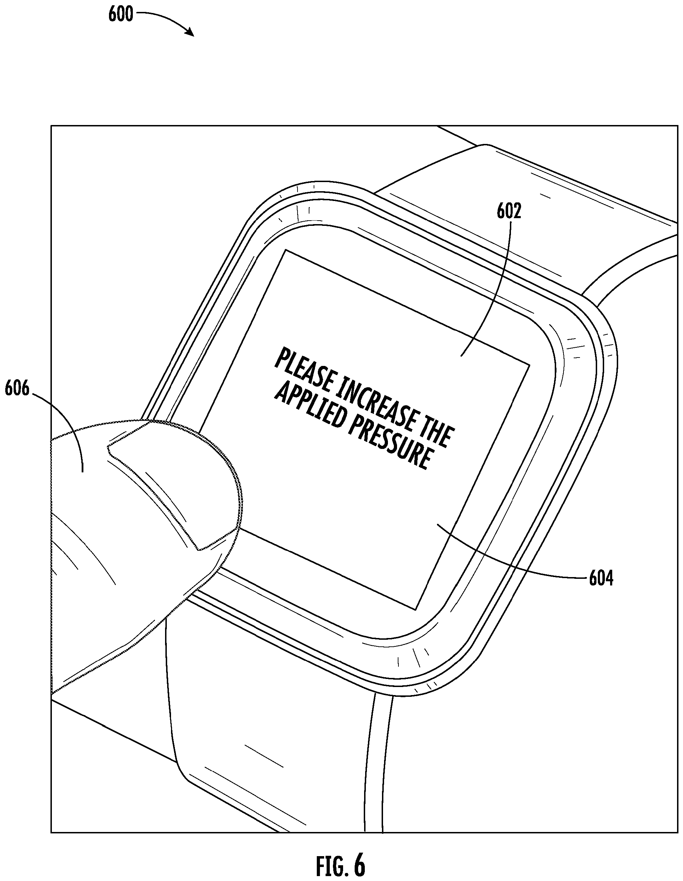

FIG. 6 illustrates an example user interface for a smartwatch 600 in accordance with example embodiments of the present disclosure. In this example, a smartwatch 600 includes a display 602. In addition the smartwatch includes one pressure sensor 606. In response to directions from the smartwatch, the user can press on the pressure sensor 606. In accordance with the determination that the user has initially applied a first level of pressure and then during the measuring period the user decreases the level of pressure to a second level of pressure, the smartwatch 600 can determine that the user should apply additional pressure. In accordance with this determination, the smartwatch can display a prompt 604 in the display 602 directing the user to increase the amount of pressure applied.

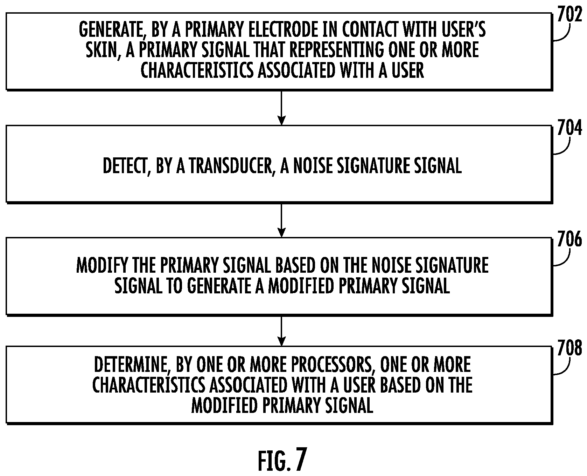

FIG. 7 is a flowchart depicting an example process of mitigating noise in a user computing device in accordance with example embodiments of the present disclosure. One or more portion(s) of the method can be implemented by one or more computing devices such as, for example, the computing devices described herein. Moreover, one or more portion(s) of the method can be implemented as an algorithm on the hardware components of the device(s) described herein. FIG. 7 depicts elements performed in a particular order for purposes of illustration and discussion. Those of ordinary skill in the art, using the disclosures provided herein, will understand that the elements of any of the methods discussed herein can be adapted, rearranged, expanded, omitted, combined, and/or modified in various ways without deviating from the scope of the present disclosure, the scope of the invention being defined by the appended claims. The method can be implemented by one or more computing devices, such as one or more of the computing devices depicted in FIGS. 1-3.

A user computing device (e.g., user computing device 100 in FIG. 1) includes a first electrode and a transducer. In some examples, the user computing device 100, at 702, generates, by a first electrode in contact with a user's skin, a first signal that represents one or more characteristics associated with a user. The physiological signal sensor is a dry electrode in contact with a user's skin. For example, the dry electrode can be a single metal contact that, when placed in contact with the user's skin, can generate a first signal in response to one or more characteristics of the user's skin or body.

The user computing device 100 can, at 704, generate a noise signature signal via a transducer. In the context of the claimed invention, the transducer includes an audio sensor situated to detect audio signals internal to the user computing device generated by lateral movement of the user computing device along the skin of the user. In another example useful to understand the claimed invention, the transducer is a pressure sensor configured to determine variations in the pressure applied by a user.

In some further examples useful to understand the claimed invention, the transducer can be a capacitance sensor and the noise signature signal can represent changes in the area in which changes in differences in effective capacitance are detected. In some further examples useful to understand the claimed invention, the transducer is an accelerometer and the noise signature signal represents a movement of the user computing device.

The user computing device 100 can, at 706, modify the first signal based on the noise signature signal to generate a modified first signal. The first signal and the noise signature signal can be represented in one of the time domain or the frequency domain.

In some examples, modifying the first signal comprises subtracting the noise signature signal from the first signal to generate the modified first signal. In other examples, modifying the first signal can comprise determining, at a particular time, whether the noise signature signal exceeds a predetermined threshold value. In accordance with a determination that the noise signature exceeds the predetermined threshold value, the user computing device 100 can store a timestamp of the particular time. The user computing device 100 can exclude the first signal associated with the timestamp from consideration when determining one or more characteristics associated with a user based on the modified first signal.

The user computing device 100 can utilize a predetermined amount of time associated with gathering sufficient data to determine one or more characteristics associated with the user. In some examples, before the predetermined amount of time has elapsed, the user computing device can determine that the modified first signal includes data sufficient to determine one or more characteristics associated with the user. For example, if the user computing device 100 is estimating the heart rate of a user, the user computing device 100 can determine that it has enough data to estimate the heart rate of a user before the predetermined amount of time has already elapsed.

In some examples, the user computing device 100 can present, on a display associated with the user computing device 100, a notification that the user can disengage from contact with the dry electrode.

Similarly, the user computing device 100 can utilize a predetermined amount of time associated with gathering sufficient data to determine one or more characteristics associated with the user. When the predetermined amount of time has elapsed, the user computing device 100 can determine that the modified first signal does not include data sufficient to determine one or more characteristics associated with the user. The user computing device 100 can present, on a display associated with the user computing device 100, a request that the user continues to remain in contact with the dry electrode.

In some further examples useful to understand the claimed invention, the transducer can be a pressure sensor. The user computing device 100 can detect, using the transducer based on the noise signature signal, a change in pressure applied by the user. The user computing device 100 can determine that the change in pressure applied by the user exceeds a predetermined threshold. In accordance with a determination that the change in pressure applied by the user exceeds the predetermined threshold, the user computing device 100 can display a message to the user requesting the user to alter the pressure applied.

In some examples, the user computing device 100 can, at 708, determine one or more characteristics associated with a user based on the modified first signal. The one or more characteristics associated with the user include the presence of sweat on a surface of the user's skin. In some examples, the presence of sweat on the surface of the user's skin is used to estimate a stress level associated with the user.

In some examples, the one or more characteristics associated with the user can include an impedance value as measured between two points on the user's skin. The impedance value as measured between two points on the user's skin can be used to estimate a stress level associated with the user.

The technology discussed herein refers to sensors and other computer-based systems, as well as actions taken and information sent to and from such systems. One of ordinary skill in the art will recognize that the inherent flexibility of computer-based systems allows for a great variety of possible configurations, combinations, and divisions of tasks and functionality between and among components. For instance, server processes discussed herein may be implemented using a single server or multiple servers working in combination. Databases and applications may be implemented on a single system or distributed across multiple systems. Distributed components may operate sequentially or in parallel.

While the present subject matter has been described in detail with respect to specific example embodiments thereof, it will be appreciated that those skilled in the art, upon attaining an understanding of the foregoing may readily produce alterations to, variations of, and equivalents to such embodiments. Accordingly, the scope of the present disclosure is by way of example rather than by way of limitation, and the subject disclosure does not preclude inclusion of such modifications, variations and/or additions to the present subject matter as would be readily apparent to one of ordinary skill in the art, the invention being defined by the appended claims.

## Claims

1. A user computing device (100), the user computing device (100) comprising:
a housing (304);
a physiological signal sensor configured to generate a first signal relating one or more characteristics associated with a user and arranged to measure a signal from outside the housing (304);
a transducer (114) configured to generate a noise signature signal based on detection of one or more noise sources; and
one or more control circuits configured to:
modify the first signal based on the noise signature signal to generate a modified first signal; and
determine one or more characteristics associated with a user based on the modified first signal, wherein
the transducer (114) includes an audio sensor arranged within the housing (304) and situated to detect a noise source in the form of audio signals internal to the user computing device (100) generated by lateral movement of the user computing device (100) along the user's skin, **characterised in that** the physiological signal sensor is a dry electrode (110) enabled to contact a user's skin.

2. The user computing device (100) of claim 1, wherein the transducer (114) further comprises a pressure sensor enabled to determine variations in pressure applied by a user.

3. The user computing device (100) of any preceding claim, wherein modifying the first signal comprises:
subtracting, from the first signal, a signal portion corresponding to the noise signature signal to generate the modified first signal.

4. The user computing device (100) of any preceding claim, wherein the first signal and the noise signature signal are represented in one of a time domain or a frequency domain.

5. The user computing device (100) of any preceding claim, wherein the transducer (114) further includes a motion sensor and the noise signature signal further represents movement of the user computing device (100).

6. The user computing device (100) of any preceding claim, wherein the transducer (114) further includes a capacitance sensor and the noise signature signal further represents changes in an area in which changes in differences in effective capacitance are detected.

7. The user computing device (100) of any preceding claim, wherein the one or more characteristics associated with the user includes a presence of sweat on a surface of the user's skin.

8. The user computing device (100) of claim 7, wherein the presence of sweat on the surface of the user's skin is used to estimate a stress level associated with the user.

9. The user computing device (100) of any preceding claim, wherein the one or more characteristics associated with the user includes an impedance value as measured between two points on the user's skin.

10. The user computing device (100) of claim 9, wherein the user computing device is configured to estimate stress level associated with the user based on the impedance value.

11. A computer-implemented method for removing noise from a signal generated by a dry electrode, the method comprising: generating, by a dry electrode (110) in a user computing device (100), in contact with user's skin, a first signal representing one or more characteristics associated with a user and arranged to measure a signal from outside a housing (304) of the user computing device (100);
detecting, by a transducer (114), a noise signature signal;
modifying, by one or more processors (102), the first signal based on the noise signature signal to generate a modified first signal; and
determining, by one or more processors (102), one or more characteristics associated with a user based on the modified first signal, wherein
the transducer (114) includes an audio sensor arranged within the housing (304) and situated to detect a noise source in the form of audio signals internal to the user computing device (100) generated by lateral movement of the user computing device (100) along the user's skin.

12. The computer-implemented method of claim 11, wherein modifying the first signal comprises:
determining, by the one or more processors (102) at a particular time, whether the noise signature signal exceeds a predetermined threshold value;
in accordance with a determination that the noise signature signal exceeds the predetermined threshold value, storing, by the one or more processors (102), a timestamp of the particular time; and
excluding, by the one or more processors (102), the first signal associated with the timestamp from consideration when determining one or more characteristics associated with a user based on the modified first signal.

13. The computer-implemented method of claim 11 or 12, wherein the user computing device (100) includes a predetermined amount of time associated with gathering sufficient data to determine one or more characteristics associated with the user, the method further comprising:
before the predetermined amount of time has elapsed:
determining, by the one or more processors (102), that the modified first signal includes data sufficient to determine one or more characteristics associated with the user; and
presenting, by the one or more processors (102) on a display (302) associated with the user computing device (100), a notification that the user can disengage from contact with the dry electrode (110).

14. The computer-implemented method of any of claims 11 to 13, wherein the user computing device (100) includes a predetermined amount of time associated with gathering sufficient data to determine one or more characteristics associated with the user, the method further comprising:
when the predetermined amount of time has elapsed:
determining, by the one or more processors (102), that the modified first signal does not include data sufficient to determine one or more characteristics associated with the user; and
presenting, by the one or more processors (102) on a display (302) associated with the user computing device (100), a request that the user continue to remain in contact with the dry electrode (110).

## Patentansprüche

1. Benutzerrechenvorrichtung (100), wobei die Benutzerrechenvorrichtung (100) Folgendes umfasst:
ein Gehäuse (304);
einen physiologischen Signalsensor, der dazu konfiguriert ist, ein erstes Signal zu erzeugen, das sich auf eine oder mehrere einem Benutzer zugeordnete Eigenschaften bezieht, und der angeordnet ist, um ein Signal von außerhalb des Gehäuses (304) zu messen;
einen Wandler (114), der dazu konfiguriert ist, ein Rauschsignatursignal basierend auf Erkennung von einer oder mehreren Rauschquellen zu erzeugen; und
eine oder mehrere Steuerschaltungen, die konfiguriert sind zum:
Modifizieren des ersten Signals basierend auf dem Rauschsignatursignal, um ein modifiziertes erstes Signal zu erzeugen; und
Bestimmen einer oder mehrerer Eigenschaften, die einem Benutzer zugeordnet sind, basierend auf dem modifizierten ersten Signal, wobei
der Wandler (114) einen Audiodatensensor beinhaltet, der innerhalb des Gehäuses (304) angeordnet ist und gelegen um eine Rauschquelle in Form von Audiodatensignalen innerhalb der Benutzerrechenvorrichtung (100), die durch laterale Bewegung der Benutzerrechenvorrichtung (100) entlang der Benutzerhaut erzeugt werden, zu detektieren, **dadurch gekennzeichnet, dass**
der physiologische Signalsensor eine trockene Elektrode (110) ist, die in der Lage ist, die Benutzerhaut zu kontaktieren.

2. Benutzerrechenvorrichtung (100) nach Anspruch 1, wobei der Wandler (114) ferner einen Drucksensor umfasst,
der in der Lage ist, Variationen des von einem Benutzer angewendeten Drucks zu bestimmen.

3. Benutzerrechenvorrichtung (100) nach einem der vorhergehenden Ansprüche, wobei das Modifizieren des ersten Signals Folgendes umfasst:
Subtrahieren eines dem Rauschen entsprechenden Abschnitts von dem ersten Signal, um das modifizierte erste Signal zu erzeugen.

4. Benutzerrechenvorrichtung (100) nach einem der vorhergehenden Ansprüche, wobei das erste Signal und das Rauschsignatursignal entweder in einem Zeitbereich oder in einem Frequenzbereich dargestellt sind.

5. Benutzerrechenvorrichtung (100) nach einem der vorhergehenden Ansprüche, wobei der
Wandler (114) ferner einen Bewegungssensor beinhaltet und das Rauschsignatursignal ferner eine Bewegung
der Benutzerrechenvorrichtung (100) darstellt.

6. Benutzerrechenvorrichtung (100) nach einem der vorhergehenden Ansprüche, wobei der
Wandler (114) ferner einen Kapazitätssensor beinhaltet und das Rauschsignatursignal ferner
Änderungen in einer Fläche darstellt, in der Änderungen in Unterschieden in der effektiven Kapazität erkannt werden.

7. Benutzerrechenvorrichtung (100) nach einem der vorhergehenden Ansprüche, wobei die eine oder die mehreren dem Benutzer zugeordneten Eigenschaften ein Vorhandensein von Schweiß auf einer Oberfläche der Benutzerhaut beinhaltet.

8. Benutzerrechenvorrichtung (100) nach Anspruch 7, wobei das
Vorhandensein von Schweiß auf der Oberfläche der Benutzerhaut verwendet wird, um eine dem Benutzer zugeordnete Stressbelastung zu schätzen.

9. Benutzerrechenvorrichtung (100) nach einem der vorhergehenden Ansprüche, wobei die eine oder die mehreren dem Benutzer zugeordneten Eigenschaften einen Impedanzwert beinhalten, der zwischen zwei Punkten auf der Benutzerhaut gemessen wird.

10. Benutzerrechenvorrichtung (100) nach Anspruch 9, wobei die Benutzerrechenvorrichtung dazu konfiguriert ist, die dem Benutzer zugeordneten Stressbelastung basierend auf dem Impedanzwert zu schätzen.

11. Computerimplementiertes Verfahren zum Entfernen von Rauschen aus einem Signal, das durch eine trockene Elektrode erzeugt wird, wobei das Verfahren Folgendes umfasst:
Erzeugen durch eine trockene Elektrode (110) in einer Benutzerrechenvorrichtung (100), die mit der Benutzerhaut in Kontakt steht, eines ersten Signals, das ein oder mehrere einem Benutzer zugeordnete Merkmale darstellt und angeordnet ist, um ein Signal von außerhalb eines Gehäuses (304) der Benutzerrechenvorrichtung (100) zu messen;
Erkennen eines Rauschsignatursignals durch einen Wandler (114); Modifizieren des ersten Signals basierend auf dem Rauschsignatursignal, um ein modifiziertes erstes Signal zu erzeugen, durch einen oder mehrere Prozessoren (102); und
Bestimmen einer oder mehrerer Eigenschaften, die einem Benutzer zugeordnet sind, basierend auf dem modifizierten ersten Signal durch einen oder mehrere Prozessoren (102), wobei
der Wandler (114) einen Audiodatensensor beinhaltet, der innerhalb des Gehäuses (304) angeordnet ist und gelegen, um eine Rauschquelle in Form von Audiodatensignalen innerhalb der Benutzerrechenvorrichtung (100), die durch laterale Bewegung der Benutzerrechenvorrichtung (100) entlang der Benutzerhaut erzeugt werden, zu detektieren.

12. Computerimplementiertes Verfahren nach Anspruch 11, wobei das Modifizieren des ersten Signals Folgendes umfasst:
Bestimmen, ob das Rauschsignatursignal zu einem bestimmten Zeitpunkt einen vorbestimmten Schwellenwert überschreitet, durch den einen oder die mehreren Prozessoren (102);
in Übereinstimmung mit einer Bestimmung, dass das Rauschsignatursignal den vorbestimmten Schwellenwert überschreitet, Speichern eines Zeitstempels der bestimmten Zeit durch den einen oder die mehreren Prozessoren (102); und
Ausschließen des dem Zeitstempel zugeordneten ersten Signals durch den einen oder die mehreren Prozessoren (102) von der Berücksichtigung, wenn eine oder mehrere einem Benutzer zugeordnete Eigenschaften basierend auf dem modifizierten ersten Signal bestimmt werden.

13. Computerimplementiertes Verfahren nach Anspruch 11 oder 12, wobei die Benutzervorrichtung (100) eine vorbestimmte Zeitspanne beinhaltet, die dem Sammeln ausreichender Daten zum Bestimmen einer oder mehrerer dem Benutzer zugeordneter Eigenschaften zugeordnet ist, wobei das Verfahren ferner Folgendes umfasst:
bevor die vorbestimmte Zeitspanne verstrichen ist:
Bestimmen, dass das modifizierte erste Signal Daten beinhaltet, die ausreichen, um eine oder mehrere dem Benutzer zugeordnete Eigenschaften zu bestimmen, durch den einen oder die mehreren Prozessoren (102); und
Darstellen einer Benachrichtigung, dass der Benutzer den Kontakt mit der trockenen Elektrode (110) aufheben kann, durch den einen oder die mehreren Prozessoren (102) auf einer Anzeige (302), die der Benutzerrechenvorrichtung (100) zugeordnet ist.

14. Computerimplementiertes Verfahren nach einem der Ansprüche 11 bis 13, wobei die Benutzervorrichtung (100) eine vorbestimmte Zeitspanne beinhaltet, die mit Sammeln ausreichender Daten zum Bestimmen einer oder mehrerer dem Benutzer zugeordneter Eigenschaften bestimmt ist, wobei das Verfahren ferner Folgendes umfasst:
wenn die vorbestimmte Zeitspanne verstrichen ist:
Bestimmen, dass das modifizierte erste Signal nicht Daten beinhaltet, die ausreichen, um eine oder mehrere dem Benutzer zugeordnete Eigenschaften zu bestimmen, durch den einen oder die mehreren Prozessoren (102); und
Darstellen einer Anforderung, dass der Benutzer weiterhin mit der trockenen Elektrode (110) in Kontakt bleibt, durch den einen oder die mehreren Prozessoren (102) auf einer Anzeige (302), die der Rechenvorrichtung (100) des Benutzers zugeordnet ist.

## Revendications

1. Dispositif informatique utilisateur (100), le dispositif informatique utilisateur (100) comprenant :
un boîtier (304) ;
un capteur de signal physiologique configuré pour générer un premier signal relatif à une ou plusieurs caractéristiques associées à un utilisateur et agencé pour mesurer un signal provenant de l'extérieur du boîtier (304) ;
un transducteur (114) configuré pour générer un signal de signature de bruit sur la base de la détection d'une ou de plusieurs sources de bruit ; et
un ou plusieurs circuits de commande configurés pour :
modifier le premier signal sur la base du signal de signature de bruit pour générer un premier signal modifié ; et
déterminer une ou plusieurs caractéristiques associées à un utilisateur sur la base du premier signal modifié, dans lequel le transducteur (114) comporte un capteur audio agencé à l'intérieur du boîtier (304) et situé pour détecter une source de bruit sous la forme de signaux audio internes au dispositif informatique utilisateur (100) générés par un mouvement latéral du dispositif informatique utilisateur (100) le long de la peau de l'utilisateur, **caractérisé en ce que**
le capteur de signal physiologique est une électrode sèche (110) capable d'entrer en contact avec la peau d'un utilisateur.

2. Dispositif informatique utilisateur (100) selon la revendication 1, dans lequel le transducteur (114) comprend également un
capteur de pression capable de déterminer des variations d'une pression appliquée par un utilisateur.

3. Dispositif informatique utilisateur (100) selon une quelconque revendication précédente, dans lequel la modification du premier signal comprend :
la soustraction, du premier signal, d'une partie de signal correspondant au signal de signature de bruit pour générer le premier signal modifié.

4. Dispositif informatique utilisateur (100) selon une quelconque revendication précédente, dans lequel le premier signal et le signal de signature de bruit sont représentés dans l'un d'un domaine temporel ou d'un domaine fréquentiel.

5. Dispositif informatique utilisateur (100) selon une quelconque revendication précédente, dans lequel
le transducteur (114) comporte également un capteur de mouvement et le signal de signature de bruit représente également un mouvement
du dispositif informatique utilisateur (100).

6. Dispositif informatique utilisateur (100) selon une quelconque revendication précédente, dans lequel
le transducteur (114) comporte également un capteur de capacité et le signal de signature de bruit représente également
des changements dans une zone dans laquelle des changements dans des différences de capacité effective sont détectés.

7. Dispositif informatique utilisateur (100) selon une quelconque revendication précédente, dans lequel les une ou plusieurs caractéristiques associées à l'utilisateur comportent une présence de sueur sur une surface de la peau de l'utilisateur.

8. Dispositif informatique utilisateur (100) selon la revendication 7, dans lequel
la présence de sueur à la surface de la peau de l'utilisateur est utilisée pour estimer un niveau de stress associé à l'utilisateur.

9. Dispositif informatique utilisateur (100) selon une quelconque revendication précédente, dans lequel les une ou plusieurs caractéristiques associées à l'utilisateur comportent une valeur d'impédance telle que mesurée entre deux points sur la peau de l'utilisateur.

10. Dispositif informatique utilisateur (100) selon la revendication 9, dans lequel le dispositif informatique utilisateur est configuré pour estimer un niveau de stress associé à l'utilisateur sur la base de la valeur d'impédance.

11. Procédé mis en œuvre par ordinateur pour supprimer le bruit d'un signal généré par une électrode sèche, le procédé comprenant :
la génération, par une électrode sèche (110) dans un dispositif informatique utilisateur (100), en contact avec la peau de l'utilisateur, d'un premier signal représentant une ou plusieurs caractéristiques associées à un utilisateur et agencé pour mesurer un signal provenant de l'extérieur d'un boîtier (304) du dispositif informatique utilisateur (100) ;
la détection, par un transducteur (114), d'un signal de signature de bruit ;
la modification, par un ou plusieurs processeurs (102), du premier signal sur la base du signal de signature de bruit pour générer un premier signal modifié ; et
la détermination, par un ou plusieurs processeurs (102), d'une ou plusieurs caractéristiques associées à un utilisateur sur la base du premier signal modifié, dans lequel
le transducteur (114) comporte un capteur audio agencé à l'intérieur du boîtier (304) et situé pour détecter une source de bruit sous la forme de signaux audio internes au dispositif informatique utilisateur (100) générés par un mouvement latéral du dispositif informatique utilisateur (100) le long de la peau de l'utilisateur.

12. Procédé mis en œuvre par ordinateur selon la revendication 11, dans lequel la modification du premier signal comprend :
le fait de déterminer, par les un ou plusieurs processeurs (102) à un moment particulier, si le signal de signature de bruit dépasse une valeur de seuil prédéterminée ;
selon une détermination du fait que le signal de signature de bruit dépasse la valeur de seuil prédéterminée, le stockage, par les un ou plusieurs processeurs (102), d'un horodatage du moment particulier ; et
l'exclusion, par les un ou plusieurs processeurs (102), du premier signal associé à l'horodatage de la prise en compte lors de la détermination d'une ou de plusieurs caractéristiques associées à un utilisateur sur la base du premier signal modifié.

13. Procédé mis en œuvre par ordinateur selon la revendication 11 ou 12, dans lequel le dispositif informatique utilisateur (100) comporte une quantité de temps prédéterminée associée à la collecte de données suffisantes pour déterminer une ou plusieurs caractéristiques associées à l'utilisateur, le procédé comprenant également :
avant que la quantité de temps prédéterminée ne soit écoulée :
la détermination, par les un ou plusieurs processeurs (102), du fait que le premier signal modifié comporte des données suffisantes pour déterminer une ou plusieurs caractéristiques associées à l'utilisateur ; et
la présentation, par les un ou plusieurs processeurs (102) sur un écran (302) associé au dispositif informatique utilisateur (100), d'une notification indiquant que l'utilisateur peut se dégager du contact avec l'électrode sèche (110).

14. Procédé mis en œuvre par ordinateur selon l'une quelconque des revendications 11 à 13, dans lequel le dispositif informatique utilisateur (100) comporte une quantité de temps prédéterminée associée à la collecte de données suffisantes pour déterminer une ou plusieurs caractéristiques associées à l'utilisateur, le procédé comprenant également :
lorsque la quantité de temps prédéterminée s'est écoulée :
la détermination, par les un ou plusieurs processeurs (102), du fait que le premier signal modifié ne comporte pas de données suffisantes pour déterminer une ou plusieurs caractéristiques associées à l'utilisateur ; et
la présentation, par les un ou plusieurs processeurs (102) sur un écran (302) associé au dispositif informatique utilisateur (100), d'une demande pour que l'utilisateur continue à rester en contact avec l'électrode sèche (110).
